# EUROPÄISCHE PATENTSCHRIFT

(11) **EP 1 346 058 B1**
(45) Veröffentlichungstag und Bekanntmachung des Hinweises auf die Patenterteilung: **02.05.2007**
(21) Anmeldenummer: 01990586.8
(22) Anmeldetag: 21.12.2001
(51) Int. Cl.: C12N 15/861, C07K 14/47, C07K 14/075, A61K 48/00, A61K 38/17, G01N 33/53

(54) **ADENOVIRALE SYSTEME UND DEREN ANWENDUNGEN**
ADENOVIRAL SYSTEMS AND THE USES THEREOF
SYSTEMES DE VECTEURS ADENOVIRAUX ET LEURS UTILISATIONS

(30) Priorität: 28.12.2000 DE 10065504; 16.10.2001 DE 10150945
(43) Veröffentlichungstag der Anmeldung: 24.09.2003
(73) Patentinhaber: Holm, Per Sonne, 82256 Fürstenfeldbruck (DE)
(72) Erfinder: Holm, Per Sonne, 82256 Fürstenfeldbruck (DE)
(74) Vertreter: Bohmann, Armin K.
(86) Internationale Anmeldenummer: PCT/EP2001/015212
(87) Internationale Veröffentlichungsnummer: WO 2002/053711

(56) Entgegenhaltungen:
- WO-A-00/39317
- WO-A-00/56909
- WO-A-99/06576
- DE-A- 10 031 122
- DE-A- 19 929 569
- US-A- 5 856 181
- US-A- 5 871 726
- US-A- 6 110 744
- US-A- 6 140 126
- KOIKE K ET AL: "Nuclear translocation of the Y-box binding protein by ultraviolet irradiation" FEBS LETTERS, ELSEVIER SCIENCE PUBLISHERS, AMSTERDAM, NL, Bd. 417, Nr. 3, 17. November 1997 (1997-11-17), Seiten 390-394, XP004261491 ISSN: 0014-5793
- SAFAK MAHMUT ET AL: "Physical and functional interaction between the Y-box binding protein YB-1 and human polyomavirus JC virus large T antigen" JOURNAL OF VIROLOGY, THE AMERICAN SOCIETY FOR MICROBIOLOGY, US, Bd. 73, Nr. 12, Dezember 1999 (1999-12), Seiten 10146-10157, XP002177057 ISSN: 0022-538X
- YUN JEANHO ET AL: "p53 negatively regulates cdc2 transcription via the CCAAT-binding NF-Y transcription factor." JOURNAL OF BIOLOGICAL CHEMISTRY, Bd. 274, Nr. 42, 15. Oktober 1999 (1999-10-15), Seiten 29677-29682, XP002193311 ISSN: 0021-9258
- KIRN D ET AL: "ADENOVIRUS E1A MUTANTS THAT SELECTIVELY REPLICATE IN AND CAUSE ENHANCED DESTRUCTION OF CANCER CELLS IN VITRO AND IN NUDE MOUSE-HUMAN TUMOR XENOGRAFTS" CANCER GENE THERAPY, NORWALK, CT, US, Bd. 5, Nr. 6, SUPPL, November 1998 (1998-11), Seite S26 XP000982296 ISSN: 0929-1903
- HEISE ET AL: "ONYX-015, an E+B gene-attenuated adenovirus, causes tumor-specific cytolysis and antitumoral efficacy that can be augmented by standard chemotherapeutic agents" NATURE MEDICINE, NATURE PUBLISHING, CO, US, Bd. 3, Nr. 6, 1. Juni 1997 (1997-06-01), Seiten 639-645, XP002095383 ISSN: 1078-8956
- BARGOU RALF C ET AL: "Nuclear localization and increased levels of transcription factor YB-1 in primary human breast cancers are associated with intrinsic MDR1 gene expression" NATURE MEDICINE, NATURE PUBLISHING, CO, US, Bd. 3, Nr. 4, April 1997 (1997-04), Seiten 447-450, XP002160622 ISSN: 1078-8956
- RITTNER K ET AL: "Conditional repression of the E2 transcription unit in E1-E3-deleted adenovirus vectors is correleated with a strong reduction in viral DNA replication and late gene expression in vitro" JOURNAL OF VIROLOGY, THE AMERICAN SOCIETY FOR MICROBIOLOGY, US, Bd. 71, Nr. 4, April 1997 (1997-04), Seiten 3307-3311, XP002130029 ISSN: 0022-538X
- FANG B ET AL: "DIMINISHING ADENOVIRUS GENE EXPRESSION AND VIRAL REPLICATION BY PROMOTER REPLACEMENT" GENE: AN INTERNATIONAL JOURNAL ON GENES AND GENOMES, ELSEVIER SCIENCE PUBLISHERS, BARKING, GB, Bd. 71, Nr. 6, 1. Juni 1997 (1997-06-01), Seiten 4798-4803, XP002062030 ISSN: 0378-1119

## Beschreibung

Die vorliegende Erfindung betrifft Nukleinsäuren umfassend adenovirale Nukleinsäure, diese umfassende Adenoviren und deren Verwendung.

Bei der Behandlung von Tumoren werden derzeit eine Vielzahl von Therapiekonzepten verfolgt. Neben der Verwendung chirurgischer Techniken stehen dabei die Chemotherapie und die Strahlentherapie im Vordergrund. All diese Techniken sind jedoch für den Patienten mit nicht unerheblichen Nebenwirkungen verbunden.

Mit der Verwendung von replikationsselektiven onkolytischen Viren wurde eine neue Plattform für die Behandlung von Tumoren geschaffen. Dabei wird eine selektive intratumorale Replikation eines viralen Agens herbeigeführt, die in der Folge zur Virusreplikation, Lyse der infizierten Tumorzelle und Verbreitung des Virus auf benachbarte Tumorzellen führt. Infolge der Beschränkung der Replikationsfähigkeit des Virus auf Tumorzellen bleibt normales Gewebe von der Infektion und damit der Lyse durch den Virus verschont. Beispiele für derartige replikationsselektive onkolytische Viren sind Gen-attenuierte Adenovirus und Herpesviren (Martuza, R. et al. Science 252, 854-858 (1991); Fueyo, J et al. Oncogene 19, 2-12 (2000))

Ein Beispiel für einen derartigen Adenoviren ist dl 1520 (Onyx-015), der bereits erfolgreich in den klinischen Phasen I und II eingesetzt wurde (Khuri, F. et al. Nature Medicine 6, 879-885 (2000). Onyx-015 ist ein Adenovirus, bei dem das E1B 55-kDa-Gen deletiert ist. Das E1B-55kDa-Genprodukt ist beteiligt an der Inhibierung von p53, dem Transport viraler mRNA und dem Abschalten der Proteinsynthese der Wirtszelle. Die Inhibierung von p53 erfolgt dabei durch Ausbilden eines Komplexes aus p53 und dem adenoviral codierten E1B-55kDa Protein. Von p53, codiert von TP53, geht ein komplexer regulatorischer Mechanismus aus (Zambetti, G.P. et al., FASEB J, 7, 855-865), der u.a. auch dazu führt, dass eine effiziente Replikation von Viren wie Adenoviren in der Zelle unterdrückt wird. Das Gen TP 53 ist in etwa 50 % aller menschlichen Tumoren deletiert oder mutiert mit der Folge, dass es zu keiner - erwünschten - Apoptose infolge einer Chemotherapie oder einer Bestrahlungstherapie kommt und damit der Erfolg dieser Tumorbehandlungen im Normalfall unterbleibt.

DNA-Tumorviren, wie Adenoviren, treiben die infizierten Zellen in die S-Phase des Zellzyklus, um die virale DNA-Replikation zu erleichtern. Onyx-015 exprimiert das E1B-55 kDa Protein nicht und repliziert selektiv in Tumorzellen gegenüber normalen Zellen. Darüberhinaus besteht eine weitere Selektivität dahingehend, dass solche Tumoren infolge der viralen Lyse der Tumorzellen eine vergleichsweise stärkere Nekrose erfahren, die p53-defizient sind, als jene, die den p53-Wildtyp aufweisen (Khuri et al., aaO). Trotz der grundsätzlichen Wirksamkeit von Onyx-015 bei der viral induzierten Onkolyse im Falle von p53-defizienten Tumoren ist die Erfolgsrate von 15 % der behandelten Tumore sehr gering.

Ries et al. (Ries, S.J. et al. Nature Medicine 6, 1128 - 1132 (2000) haben eine prinzipielle Möglichkeit aufgezeigt, wie Onyx-015 auch bei Tumoren mit p53-Wildtyp erfolgreich verwendet werden können. Dabei wird das Tumorsuppressor-Protein p14ARF nicht exprimiert. In Folge des Fehlens von p14ARF unterbleibt die normale Reaktion des p53-Systems auf eine virale Infektion und erlaubt damit die Replikation von Onyx-015 auch in diesen Tumoren. Eine Anwendung dieser Erkenntnis setzt jedoch voraus, dass in der Tumorzelle ein geeigneter genetischer Hintergrund existiert oder durch geeignete therapeutische Maßnahmen bereitgestellt wird. Im ersteren Falle würde sich die Anzahl der mittels Onxy-015 therapierbaren Tumoren weiter verringern, im zweiten Fall wäre eine aufwendige Änderung des genetischen Hintergrundes der Tumorzellen erforderlich.

Der vorliegenden Erfindung liegt die Aufgabe zugrunde, die bestehenden adenoviralen Systeme für die viral induzierte Onkolyse zu verbessern. Insbesondere soll dabei die Erfolgsrate, mit der Tumoren erfolgreich behandelt werden können, erhöht sein verglichen mit dem Stand der Technik.

Es ist eine weitere Aufgabe der vorliegenden Erfindung, adenovirale Systeme für die viral induzierte Onkolyse bereitzustellen, die auch bei solchen Tumoren wirksam sind, die vom p53-Wildtyp sind.

Erfindungsgemäß wird die Aufgabe durch den Gegenstand der unabhängigen Ansprüche gelöst. Bevorzugte Ausführungsformen ergeben sich aus den Unteransprüchen.

Im Zusammenhang mit den hierin offenbarten Nukleinsäuren wird der Begriff auch im Sinne von Nukleinsäuresequenzen verwendet. Bei den erfindungsgemäßen Nukleinsäuren ebenso wie bei den erfindungsgemäßen Adenoviren handelt es sich bevorzugterweise um rekombinante Produkte, insbesondere dann, wenn eine Veränderung gegenüber dem Wildtyp vorgenommen wurde. Im Zusammenhang mit der vorliegenden Erfindung weisen die erfindungsgemäßen Nukleinsäuren sowie die erfindungsgemäßen Adenoviren typischerweise eine E1-Deletion, eine E1-E3-Deletion und/oder eine E4-Deletion auf, d. h. die Nukleinsäure bzw. die entsprechenden Adenoviren sind nicht in der Lage, funktional aktive E1- und/oder E3- und/oder E4-Expressionsprodukte zu erzeugen bzw. entsprechende Produkte können von diesen nicht erzeugt werden. Typischerweise wird dies durch eine Deletion oder eine entsprechende Mutation, einschließlich einer Punktmutation, bewerkstelligt.

In einer Ausführungsform ist vorgesehen, dass ein oder mehrere der Proteine der E1A-Region transaktivierend auf die adenovirale Genexpression, aber nicht auf die Replikation eines Adenovirus aktivierend wirkt/wirken.

Der Erfindung liegt die überraschende Erkenntnis zugrunde, dass nach Infektion einer Zelle, typischerweise einer Tumorzelle mit Adenovirus eine Komplexbildung zwischen YB-1 und dem adenoviralen Genprodukt E1B-55 kDa und infolge dieser Komplexbildung ein Transport von YB-1 in den Kern erfolgt, was eine effektive Replikation des Virus im Zellkern in vivo erlaubt. Dabei wurde weiter festgestellt, dass auch E4orf6 an E1B-55 K bindet (Weigel, S., Dobbelstein, M. J. Virology, 74, 764-772, 2000, The nuclear export signal within the E4orf6 protein of adenovirus type 5 supports virus replication and cytoplasmic accumulation of viral mRNA; Keith N. Leppard, Seminars in Virology, 8, 301-307, 1998. Regulated RNA processing and RNA transport during adenovirus infection) und somit den Transport bzw. die Verteilung von E1B-55 K in den Kern vermittelt. Durch das Zusammenwirken von E1B-55 K und YB-1 bzw. E1B-55 K, YB-1 und E4orf6 erfolgt somit eine effiziente Replikation des Virus, was wiederum zur einer Lyse der Zelle, Freisetzung des Virus und Infektion und Lyse benachbarter Zellen führt, so dass im Falle der Infektion einer Tumorzelle bzw. eines Tumors letztlich eine Lyse des Tumors, d.h. eine Onkolyse, auftritt.

Eine weitere der Erfindung zugrundeliegende Erkenntnis besteht darin, dass YB-1 als Transkriptionsfaktor an den späten E2-Promotor (engl. E2 late Promotor) von Adenovirus bindet und die Replikation des Adenovirus über diesen aktiviert. Damit werden neue Adenoviren und adenovirale Systeme für die Onkolyse möglich.

Weiterhin liegt der vorliegenden Erfindung die überraschende Erkenntnis zugrunde, dass die Expression des Transgens YB-1 in einem adenoviralen Vektor zur viralen Replikation führt. Dabei werden die viralen Gene E1B, E3 und E4 nicht eingeschaltet, was im wesentlichen darin begründet liegt, dass der adenovirale Vektor hinsichtlich E1 und/oder E3 deletiert ist. Diese Gene sind jedoch für eine sehr effiziente Replikation bzw. Partikelbildung notwendig (Goodrum, F. D., Ornelles, D. A. Roles for the E4 orf6, orf3, and E1B 55-kilodalton proteins in cell cycleindependent adenovirus replication. J. Virol., 73, 74474-7488 (1999); Medghalchi, S., Padmanabhan, R., Ketner, G. Early region 4 modulates adenovirus DNA replication by two genetically separable mechanisms. Virology, **236**, 8-17 (1997). Es ist weiterhin bekannt, dass zwei Proteine (das 12S- und das 13S-Protein), die von E1A codiert werden, die Expression der anderen adenoviralen Gene steuern bzw. induzieren (Nevins, J. R. Mechanism of activation of early viral transcription by the adenovirus E1A gene products. Cell 26, 213-220 (1981); Boulanger, P et al. (1991); Biochem. J. 275, 281-299). Dabei hat sich gezeigt, dass hauptsächlich die CR3-Region des 13S-Proteins die transaktivierende Funktion ausübt (Wong HK, Ziff EB. Complementary functions of Ela conserved region 1 cooperate with conserved region 3 to activate adenovirus serotype 5 early promoters. J Virol. 1994, 68(8):4910-20). Adenoviren, die bestimmte Deletionen in der CR1- und/oder CR2-Region des 13S-Proteins aufweisen, können nicht replizieren, wirken aber noch transaktivierend auf die viralen Gene bzw. Promotoren (Wong HK, Ziff EB. Complementary functions of Ela conserved region 1 cooperate with conserved region 3 to activate adenovirus serotype 5 early promoters. J Virol. 1994, 68(8):4910-20).

Die Kombination eines derartigen Systems, d. h. eines Systems, welches die viralen Gene einschaltet, aber nicht zur viralen Replikation fähig ist, mit einer Tumor- bzw. Gewebespezifischen Expression des Transgens YB-1 würde hingegen eine sehr effektive virale Replikation bzw. Partikelbildung und damit eine Onkolyse erlauben. Als geeignete Tumor- bzw. Gewebe-spezifische Promotoren können jene verwendet werden, wie sie hierin insgesamt beschrieben wurden.

YB-1 ist ein Vertreter der Y-Box-Proteinfamilie, die an das DNA-Sequenzmotiv Y-Box bindet. Das Y-Box-Motiv stellt ein transkriptionell regulatorisches Element dar, das sich in den Promotor-oder Enhancer-Regionen einer Anzahl unterschiedlicher Gene findet, die ein Rolle bei der Regulation der Zellproliferation spielen (Ladomery, M. et al., 1995; Bioassays 17: 9-11; Didier, D.K. et al., 1988, PNAS, 85, 7322-7326).

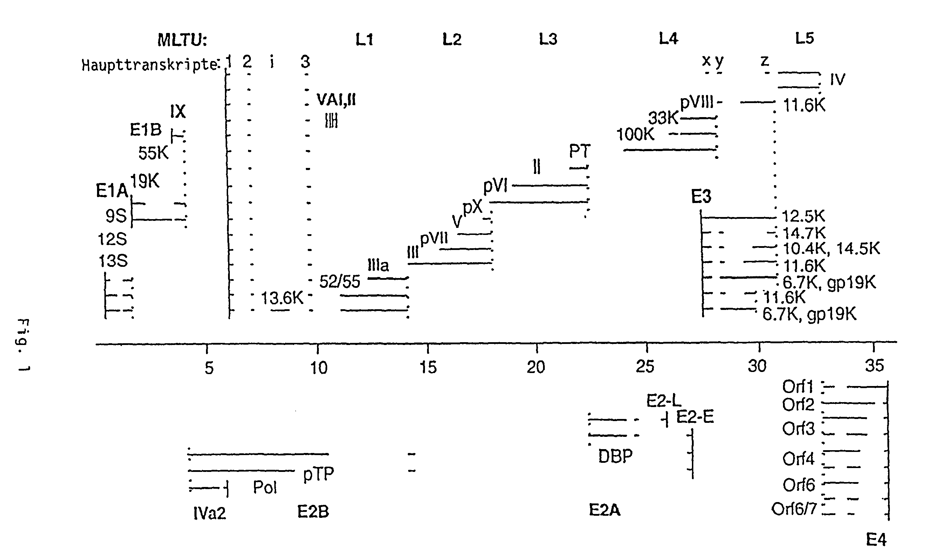

Adenoviren sind in der Technik bekannt. Dabei handelt es sich um dsDNA-Viren (Boulanger, P et al. (1991); Biochem. J. 275, 281-299). Die Organisation des Genoms ist in Fig. 1 dargestellt. Die vollständige Nukleotidsequenz des adenoviralen Genoms ist bekannt und beschrieben in (Chroboczek, J. et al., Virology 1992, 186, 280-285). Ein für die Verwendung von Adenoviren besonders wichtiger Teil des Genoms sind die sogenannten frühen oder early Gene und deren Genprodukte, die als E1, E2, E3 und E4 bezeichnet werden. Dabei umfasst E1 zwei Genprodukten E1A und E1B, die Onkogene darstellen. Die insgesamt drei Genprodukte der Gruppe E2 sind zusammen mit den Genprodukten E3 und E4 an der Replikation beteiligt.

Die im Stand der Technik bekannten adenoviralen Systeme zur Onkolyse wie beispielsweise Onyx-015 weisen eine Deletion für E1B - 55 kDa-Protein auf. Diese Deletion war vorgenommen worden unter der Annahme, dass ein intaktes p53-Gen einer effiziente Replikation in vivo entgegenwirkt und um eine adenovirale Replikation in vivo nur in p53-negativen/mutierten Zellen sicher zu stellen, führt jedoch verglichen mit dem Wildtyp zu einer um zwei Größenordnungen verringerten Partikelanzahl infolge gestörter Replikation. Andererseits greifen diese adenoviralen Systeme nach dem Stand der Technik auf E1A zurück, um vermittels des frühen E2-Promotors (E2 early Promotor) die in vivo Replikation zu steuern.

Die vorliegende Erfindung wendet sich von diesem Prinzip insoweit ab, als dass die hierin beschriebenen adenovirale Systeme auf den späten E2-Promotor zurückgreifen.

Im Rahmen der vorliegenden Erfindung sollen hierin die Begriffe Adenovirus und adenovirale Systeme als im wesentlichen die gleiche Bedeutung aufweisend verstanden werden. Unter Adenovirus soll dabei insbesondere das vollständige Viruspartikel verstanden werden umfassend das Kapsid und die Nukleinsäure. Der Begriff adenovirales System stellt insbesondere darauf ab, dass die Nukleinsäure gegenüber dem Wildtyp verändert ist. Bevorzugt umfassen derartige Änderungen Änderungen im Aufbau des Genoms des Adenovirus wie Deletieren und/oder Hinzufügen und/oder Mutieren von Promotoren, regulativen Sequenzen und codierende Sequenzen (wie Leserahmen). Der Begriff adenovirale Systeme wird darüber hinaus bevorzugt in dem Zusammenhang verwendet, dass es sich dabei um einen Vektor handelt.

Die adenoviralen Nukleinsäuren, auf die hierin Bezug genommen wird, sind im Stand der Technik bekannt. Es ist im Rahmen der Kenntnisse der Fachleute auf diesem Gebiet, für die Erfindung unwesentlichen adenoviralen Nukleinsäuresequenzen zu deletieren bzw. zu mutieren. Derartige Deletionen können z. B die für E3 codierende Nukleinsäure betreffen. In bevorzugten Ausführungsformen können diese adenoviralen Nukleinsäuren noch in das virale Kapsid verpackt werden und damit infektiöse Partikel ausbilden. Gleiches gilt für die erfindungsgemäßen Nukleinsäuren. Generell gilt es auch noch festzuhalten, dass die adenoviralen Systeme hinsichtlich einzelner oder mehrerer Expressionsprodukte defizient sein können. Dabei ist zu berücksichtigen, dass dies zum einen darauf beruhen kann, dass die für die das Expressionsprodukt codierende Nukleinsäure vollständig oder in dem Maße mutiert oder deletiert ist, dass im wesentlichen kein Expressionsprodukt mehr gebildet wird, oder darauf, dass regulative bzw. die Expression steuernde Elemente wie Promotoren oder Transkriptionsfaktoren fehlen, sei es auf der Ebene der Nukleinsäure (Fehlen eines Promotors; cis-wirkende Element) oder auf der Ebene des Translations- bzw. Transkriptionssystems (trans-wirkende Elemente). Gerade der letztere Aspekt kann dabei vom jeweiligen zellulären Hintergrund abhängen.

Bei der erfindungsgemäßen Nukleinsäure, die eine adenovirale Nukleinsäure und zusätzlich eine für YB-1 codierende Nukleinsäuresequenz umfasst, handelt es sich bevorzugterweise um eine rekombinante Nukleinsäuren. Der Leserahmen für die für YB-1 codierende Nukleinsäure kann dabei unter der Kontrolle eines die Expression und/oder Translation steuernden Elementes stehen. Dabei kann es sich beispielsweise um einen adenoviralen Promotor oder einen nichtadenoviralen Promotors handeln. Geeignete nicht-adenovirale Promotoren können ausgewählt sein aus der Gruppe, die Cytomegalovirus-Promotor, RSV-(Rous sarcoma Virus) - Promotor, adenovirus-basierender Promotor Va I und den nicht-viralen YB-1-Promotor umfasst. Weitere virale Promotoren, die im Zusammenhang mit einem jeden Aspekt der hierin offenbarten Erfindung verwendet werden können, stellen der Telomerase-Promotor, der Alpha-Fetoprotein (AFP)-Promotor, der Caecinoembryonic Antigen Promotor (CEA) (Cao, G., Kuriyama, S., Gao, J., Mitoro, A., Cui, L., Nakatani, T., Zhang, X., Kikukawa, M., Pan, X., Fukui, H., Qi, Z. Caomparison of carcinoembryonic antigen promoter regions isolated from human colorectal carcinoma and normal adj acent mucosa to induce strong tumour-selective gene expression. Int. J. Cancer, 78, 242-247, 1998), der L-Plastin-Promotor (Chung, I., Schwartz, PE., Crystal, RC., Pizzorno, G, Leavitt, J., Deisseroth, AB. Use of L-plastin promoter to develop an adenoviral system that confers transgene expression in ovarian cancer cells but not in normal mesothelial cells. Cancer Gene Therapy, 6, 99-106, 1999), Argenin-Vasopressin-Promotor (Coulson, JM, Staley, J., Woll, PJ. Tumour-specific arginine vasopressin promoter activation in small-cell lung cancer. British J. Cancer, 80, 1935-1944, 1999) und der PSA-Promotor (Hallenbeck PL, Chang, YN, Hay, C, Golightly, D., Stewart, D., Lin, J., Phipps, S., Chiang, YL. A novel tumour-specific replication-restricted adenoviral vector for gene therapy of hepatocellular carcinoma. Human Gene Therapy, 10, 1721-1733, 1999) dar.

Hinsichtlich des Telomerase-Promotors ist bekannt, dass dieser in humanen Zellen von einer zentralen Bedeutung ist. So wird die Telomeraseaktivität durch die transkriptionelle Kontrolle des Telomerase reverse Transkriptase-Gens (hTERT), welches die katalytische Untereinheit des Enzyms darstellt, reguliert. Die Expression der Telomerase ist in 85% von humanen Tumorzellen aktiv. Dahingegen ist sie in den meisten normalen Zellen inaktiv. Ausgenommen sind Keimzellen und embryonales Gewebe [Braunstein, I. et al. (2001). Human telomerase reverse transcription promoter regulation in normal and malignant human ovarian epithelial cells. Cancer Research, 61, 5529-5536; Majumdar AS et al. (2001). Tehe telomerase reverse transcriptase promoter drives effcacious tumor suicide gene therapy while preventing hepatotoxicity encountered with constitutive promoters. Gene Therapy, 8, 568-578]. Genaue Untersuchungen am hTERT-Promotor haben gezeigt, dass Fragmente des Promotors von 283 bp bzw. 82 bp vom Initiationscodon entfernt für eine spezifische Expression in Tumorzellen ausreichend sind (Braunstein I. et al.; Majumdar AS et al., aaO). Daher eignet sich dieser Promotor bzw. die spezifischen Fragmente, um eine spezifische Expression eines Transgens nur in Tumorzellen zu erzielen. Der Promotor soll die Expression des Transgens YB-1 und/oder ein verkürzte Form, die funktionell noch aktiv ist, nur in Tumorzellen ermöglichen. Die Expression des Transgens in einem adenoviralen Vektor führt dann zur viralen Replikation des adenoviralen Vektors und in der Folge zu einer Onkolyse. Es ist auch im Rahmen der vorliegenden Erfindung, dass der Leserahmen des YB-1 im Leserahmen (engl. "in frame") mit einem oder mehreren der Genprodukte des adenoviralen Systems ist. Der Leserahmen von YB-1 kann jedoch auch unabhängig davon sein. Bei der für YB-1 codierenden Nukleinsäure kann es sich um die vollständige Sequenz handeln. Es ist jedoch auch im Rahmen der vorliegenden Erfindung, dass die Nukleinsäuresequenz verkürzt ist. Eine derartige verkürzte Nukleinsäuresequenz und damit auch ein derartiges verkürztes YB-1-Protein ist insbesondere auch dann noch im Rahmen der vorliegenden Erfindung, wenn ein derartiges verkürztes YB-1 noch eine Funktion oder Eigenschaft aufweist, wie das vollständige YB-1. Eine derartige Funktion oder Eigenschaft ist beispielsweise die Fähigkeit, in den Kern zu gelangen, mit oder ohne an E1B-55kDa-Protein zu binden, oder an den E2 late- Promotor zu binden.

Die adenovirale Nukleinsäure, die in der erfindungsgemäßen Nukleinsäure umfasst ist, kann dabei eine jede adenovirale Nukleinsäure sein, die zu einem Replikationsereignis für sich oder in Verbindung mit weiteren Nukleinsäuresequenzen führt. Bei den weiteren Nukleinsäuresequenzen kann es sich beispielsweise um YB-1 handeln. Dabei ist es möglich, wie hierin noch ausgeführt werden wird, dass mittels Helferviren die für die Replikation erforderlichen Sequenzen und/oder Genprodukte bereitgestellt werden. Ein Beispiel für eine derartige adenovirale Nukleinsäure stelle die Nukleinsäure von Onyx-015 dar, die eine Expression von E1A aber nicht für E1B erlaubt.

Bei der Ausführungsform der erfindungsgemäßen Nukleinsäure, die eine für YB-1 codierende Nukleinsäuresequenz umfasst, kann vorgesehen sein, dass die adenovirale Nukleinsäure eine für E1-B codierende Nukleinsäure umfasst. Dabei ist es möglich, dass die für E1-B codierende Nukleinsäure zwar vorhanden ist, jedoch das durch sie codierte E1-B nicht exprimiert wird. Dies wird beispielsweise dadurch bewerkstelligt, dass der für E1-B codierenden Nukleinsäure ein geeigneter Promotor fehlt. Dies kann beispielsweise dann der Fall sein, wenn E1A nicht exprimiert wird. Es ist jedoch auch im Rahmen der vorliegenden Erfindung, dass es zu einer Expression von E1-B kommt, beispielsweise indem die für E1-B codierende Nukleinsäure unter der Kontrolle eines geeigneten Promotors steht. Ein geeigneter Promotor ist beispielsweise ausgewählt aus der Gruppe, die Cytomegalovirus-Promotor, RSV-(Rous sarcoma Virus) - Promotor, adenovirus-basierender Promotor Va I und den nicht-viralen YB-1-Promotor umfasst. Darüber hinaus eignen sich auch in diesem Falle die vorstehend genannten Promotoren, d. h. der Telomerase-Promotor, der Alpha-Fetoprotein (AFP)-Promotor, der Caecinoembryonic Antigen Promotor (CEA), der L-Plastin-Promotor, Argenin-Vasopressin-Promotor und der PSA-Promotor.

Wie hierin offenbart ist E1-B-55k an der Verteilung bzw. dem Transport von YB-1 in den Zellkern beteiligt. Da E4orf6 wieder rum an E1-B-55k bindet und ebenfalls für den Transport von E1-B-55 in den Kern verantwortlich ist, spielt insoweit E4orf6 auch eine bedeutende Rolle für die Verteilung bzw. den Transport von YB-1 in den Zellkern. Es ist somit auch im Rahmen der vorliegenden Erfindung, dass die Gene der E4-Region, insbesondere E4orf6 auch unter Kontrolle eines der vorstehend genannten Promotors steht. In einer bevorzugten Ausführungsform ist dabei vorgesehen, dass der adenovirale E4-Promotor nicht mehr funktional ist. Es ist dabei besonders bevorzugt, wenn der adenovirale E4-Promotor deletiert ist.

Bei einer Ausführungsform der erfindungsgemäßen Nukleinsäure, die eine für YB-1 codierende Nukleinsäuresequenz umfasst, kann vorgesehen sein, dass die adenovirale Nukleinsäure eine für E1-A codierende Nukleinsäure umfasst, die adenovirale Nukleinsäure jedoch keine Expression von E1-B erlaubt und somit dem Aufbau der DNA von Onyx-015 entspricht.

Allgemein gilt hierin, dass wann immer Bezug genommen wird auf E1-B, dies bevorzugterweise für E1B 55 kDa-Protein gilt, sofern sich kein hierzu gegenteiliger Hinweis findet.

Sofern hierin auf codierende Nukleinsäuresequenzen Bezug genommen wird und es sich dabei um solche Nukleinsäuresequenzen handelt, die bekannt sind, ist es im Rahmen der Erfindung, dass nicht nur die identische Sequenz verwendet wird, sondern auch hiervon abgeleitete Sequenzen. Unter abgeleiteten Sequenzen sollen hierin insbesondere solche Sequenzen verstanden sein, die noch zu einem Genprodukt führen, das eine Funktion aufweist, die einer Funktion der nicht abgeleiteten Sequenz entspricht. Dies kann durch einfache Routinetests festgestellt werden. Ein Beispiel für derartige abgeleitete Nukleinsäuresequenzen sind jene Nukleinsäuresequenzen, die für das gleiche Genprodukt, insbesondere für die gleiche Aminosäuresequenz codieren, jedoch infolge der Degeneriertheit des genetischen Codes eine andere Basenabfolge aufweisen.

Ein weiterer Aspekt bestehend in der erfindungsgemäßen Nukleinsäure umfassend eine für YB-1 codierende Nukleinsäure und eine einen Kerntransport von YB-1 vermittelnde Nukleinsäuresequenz beruht auf der ebenfalls überraschenden Erkenntnis, dass bei Vorhandensein von YB-1 im Kern, insbesondere unabhängig vom Zellzyklus, eine Replikation von Adenoviren in einer Zelle, bevorzugterweise in einer Tumorzelle erfolgt. Als Adenoviren bzw. adenovirale Systeme und damit die entsprechenden Nukleinsäuren können im Zusammenhang damit und in Kombination mit diesen erfindungsgemäßen Nukleinsäuren die erfindungsgemäßen Nukleinsäuren, Adenoviren und adenoviralen Systemesowie die im Stand der Technik bekannten Adenoviren wie beispielsweise Onyx-015 verwendet werden. Geeignete den Kerntransport vermittelnde Nukleinsäuresequenzen sind den Fachleuten auf dem Gebiet bekannt und beispielsweise beschrieben in (Whittaker, G.R. et al., Virology, 246, 1-23, 1998; Friedberg, E.C., TIBS 17, 347, 1992; Jans, D.A. et al., Bioessays 2000 Jun; 22(6): 532-44; Yoneda, Y., J. Biocehm. (Tokyo) 1997 May; 121(5): 811-7; Boulikas, T., Crit Rev. Eukaryot. Gene Expr. 1993; 3(3): 193-227). Bei den den Kerntransport vermittelnden Nukleinsäuresequenzen können verschiedene Prinzipien verwendet werden. Ein derartiges Prinzip besteht darin, dass, dass YB-1 als Fusionsprotein mit einem Signalpeptid ausgebildet wird und infolge des Signalpeptids YB-1 in den Zellkern geschleust wird. Ein weiteres Prinzip besteht darin, dass YB-1 mit einer Transportsequenz versehen wird, die dazu führt, dass YB-1, bevorzugterweise ausgehend von einer Synthese im Cytoplasma, in den Zellkern geschleust wird und dort die virale Replikation befördert. Ein Beispiel für eine besonders wirksame den Kerntransport vermittelnde Nukleinsäuresequenz stellt die TAT-Sequenz von HIV dar, die neben weiteren geeigneten derartigen Nukleinsäuresequenzen beispielsweise beschrieben ist in Efthymiadis, A., Briggs, LJ, Jans, DA. The HIV-1 tat nuclear localisation sequence confers novel nuclear import properties. JBC, 273, 1623.

Ein weiterer Aspekt der Erfindung bestehend in einer erfindungsgemäßen Nukleinsäure umfassend eine adenovirale Nukleinsäure, wobei die adenovirale Nukleinsäure anstelle des späten (late) E2-Promotors einen tumorspezifischen Promotor aufweist, beruht ebenfalls auf der überraschenden Erkenntnis, dass die Expression und somit die Replikation des Adenovirus und damit die Onkolyse wesentlich von der Steuerung der adenoviralen Gene und Genprodukte, insbesondere der E2-Region durch den späten E2-Promotor, insbesondere in vivo, abhängt. Die eine Transkriptionseinheit darstellende E2-Region von Adenovirus besteht aus den E2A- und E2B-Genen, die für die virale Replikation vitale Proteine codieren: pTP (precursor terminal proein), die DNA-Polymerase und DBP, ein multifunktionelles DNA-Bindungs-Protein. Im Rahmen der vorliegenden Erfindung bezeichnen die Begrifflichkeiten deletierter Promotor, nicht-funktional aktiver, funktional nicht aktiver oder nicht-funktionaler Promotor, dass der Promotor als solcher nicht mehr aktiv ist, d. h. von diesem aus keine Transkription mehr erfolgt. Ein derartiger nicht-funktionaler Promotor kann auf den Fachleuten auf diesem Gebiet bekannten Wegen erzeugt werden. Beispielsweise kann dies erfolgen durch eine vollständige Deletion des Promotors, durch eine teilweise Deletion des Promotors oder aber auch durch eine Punktmutation. Weitere Möglichkeiten der Mutationen derartiger Promotoren können darin bestehen, dass die räumliche Beziehung der den Promotor aufbauenden Elemente verändert und dieser dadurch funktional inaktiv wird.

Mit der Inaktivierung des späten E2-Promotors und dessen Ersatz durch einen tumorspezifischen bzw. gewebespezifischen Promotor und damit dem Herstellen einer der erfindungsgemäßen Nukleinsäuren wird gewährleistet, dass die für die adenovirale Replikation wichtigen Gene bzw. Genprodukte unter der Kontrolle des Tumors bzw. des entsprechenden Gewebes stehen mit der Folge, dass die Replikation des Adenovirus spezifisch im Tumor oder in einem bestimmten Gewebe erfolgt. Damit wird der Forderung nach spezifischer viral vermittelter Lyse von Zellen entsprochen und damit die Sicherheit des Systems gewährleistet. Die Inaktivierung des späten E2-Promotors kann dabei dadurch erfolgen, dass dieser vollständig deletiert ist. Es ist jedoch auch im Rahmen der Erfindung, dass der späte E2-Promotor so verändert ist, dass er nicht mehr als Promotor funktionell aktiv ist. Dies kann beispielsweise dadurch bewerkstelligt werden, dass die Bindungsstelle des Promotors für YB-1 so verändert, beispielsweise mutiert oder deletiert, wird, dass eine Bindung von YB-1 an den Promotor nicht mehr möglich wird. Eine entsprechende Deletion könnte die Deletion der Y-Box aus dem Promotor darstellen. Der hierin verwendete Begriff, dass eine erfindungsgemäße Nukleinsäure anstelle des E2-Promotors einen gewebe- oder tumorspezifischen Promotor aufweist, umfasst die vorstehend beschriebenen Möglichkeiten. In so weit ist der Begriff funktional zu verstehen.

Um die Sicherheit des Systems, welches die Expression der E2-Genprodukte über einen Tumor- bzw. Gewebe-spezifischen Promotor steuert, nochmals zu verbessern, soll in einer bevorzugten Ausführungsform gleichzeitig zum E2 late Promotor der E2 early Promotor funktional inaktiv sein, beispielsweise deletiert bzw. so verändert werden, dass er nicht mehr als Promotor funktional aktiv ist. Damit wird sichergestellt, dass der E2 early Promotor keinen Einfluss auf die Expression der E2-Gene ausüben kann. Stattdessen werden erfindungsgemäß die beiden Promotoren, d. h. der E2 late Promotor sowie der E2 early Promotor bevorzugter Weise durch einen Gewebe- oder Tumor-spezifischen Promotor ersetzt. Auch hier können die hierin im Zusammenhang mit der YB-1 kodierenden Nukleinsäuresequenz beschriebenen Promotoren verwendet werden.
Die adenovirale Nukleinsäure bzw. die entsprechenden Adenoviren umfassen bevorzugterweise die Gene für E1A, E1B, E2 und E3. In bevorzugten Ausführungsformen kann die für E3 codierende Nukleinsäure deletiert sein.

Die vorstehend beschriebenen Konstrukte von Adenoviren und insbesondere deren Nukleinsäuren können auch in eine Zelle, insbesondere eine Tumorzelle, in Teilen eingebracht werden, wobei dann infolge der Anwesenheit der verschiedenen Einzelkomponenten diese so zusammenwirken, als stammten die Einzelkomponenten von einer einzelnen Nukleinsäure. Eine typische Ausprägung dieses hierin als adenovirales Replikationssystem bezeichneten sieht dabei vor, dass die adenovirale Nukleinsäure defizient für die Expression des E1A-Proteins ist. Das bevorzugterweise zelluläre Replikationssystem umfasst eine Nukleinsäure eines Helfervirus, wobei die Nukleinsäure des Helfervirus eine Nukleinsäuresequenz umfasst, die für YB-1 codiert. Dabei kann vorgesehen sein, dass die adenovirale Nukleinsäure oder die Nukleinsäure des Helfervirus einzeln oder getrennt als replizierbare Vektoren vorliegen.

Die erfindungsgemäßen Nukleinsäuren können als Vektoren vorliegen. Bevorzugterweise handelt es sich um virale Vektoren. Im Falle der adenovirale Nukleinsäuren umfassenden erfindungsgemäßen Nukleinsäuren ist das Viruspartikel dabei der Vektor. Es ist jedoch auch im Rahmen der vorliegenden Erfindung, dass die erfindungsgemäßen Nukleinsäuren in einem Plasmidvektor vorliegen. In einem jeden Fall weist der Vektor Elemente auf, die für die Vermehrung der inserierten Nukleinsäure (Replikation) und ggf. Expression der inserierten Nukleinsäure sorgen bzw. diese steuern. Geeignete Vektoren, insbesondere auch Expressionsvektoren, und Elemente sind den Fachleuten auf dem Gebiet bekannt und beispielsweise beschrieben in Grunhaus, A., Horwitz, M.S., 1994, Adenoviruses as cloning vectors. In Rice, C., Hrsg., Seminars in virology, London: Saunders Scientific Publications, 1992; 237 - 252.

Die oben beschriebene Ausführungsform, dass die verschiedenen Elemente der erfindungsgemäßen Nukleinsäure nicht notwendigerweise auf nur einem Vektor enthalten sein müssen, trägt der Aspekt der Erfindung Rechnung, der die Vektorgruppe umfasst. Eine Vektorgruppe umfasst entsprechend mindestens zwei Vektoren. Ansonsten gilt betreffend die Vektoren das hierin allgemein zu Vektoren ausgeführte.

Die erfindungsgemäßen Adenoviren sind durch die verschiedenen hierin offenbarten Nukleinsäuren charakterisiert und umfassen ansonsten all jene den Fachleuten auf dem Gebiet bekannten Elemente, wie dies auch bei Adenoviren vom Wildtyp der Fall ist (Shenk, T.: Adenoviridae: The virus and their replication. Fields Virology, 3. Auflage, Hrsg. Fields, B.N., Knipe, D.M., Howley, P.M. et al., Lippincott-Raven Publishers, Philadelphia, 1996, Kapitel 67).

Die erfindungsgemäßen Agenzien, d.h. die Nukleinsäuren, diese enthaltende Vektoren und Vektorengruppen, Zellen und Adenoviren und adenoviralen Replikationssysteme können für die Herstellung eines Medikamentes verwendet werden. Infolge der spezifischen Aktivitäten der erfindungsgemäßen Agenzien wird das Medikament bevorzugt ein solches für die Behandlung und/oder Prophylaxe von Tumorerkrankungen sein. Grundsätzlich eignen sich diese Agenzien für alle Tumorerkrankungen bzw. Tumoren, insbesondere auch solche Tumoren, die p53 enthalten, und solche, denen p53 fehlt. Unter dem Begriff Tumor sollen sowohl maligne wie benigne Tumoren verstanden werden.

Das Medikament kann dabei in verschiedenen Formulierungen vorliegen, bevorzugterweise in einer flüssigen Form. Weiterhin wird das Medikament Hilfsstoffe wie Stabilisatoren, Puffer, Konservierungsstoffe und dergleichen enthalten, die dem Fachmann auf dem Gebiet der Galenik bekannt sind.

Das Medikament kann dabei insbesondere auch weitere pharmazeutisch aktive Verbindungen enthalten. Die Art und der Umfang dieser weiteren pharmazeutisch aktiven Verbindungen wird dabei von der Art der Indikation abhängen, für die das Medikament eingesetzt wird. Im Falle der Verwendung des Medikamentes für die Behandlung und/oder die Prophylaxe von Tumorerkrankungen werden typischerweise Zytostatika, wie beispielsweise cis-Platin und Taxol, Daunoblastin, Adriamycin und/oder Mitoxantron verwendet.

Die Verwendung von attenuierten Adenoviren wie Onxy-015, der hinsichtlich des E1B-55 kDa-Proteins defizient ist, ist bei der Verwendung zur viralen Onkolyse mit vergleichsweise geringen Erfolgsaussichten verbunden. Der vorliegende Erfinder hat überraschenderweise festgestellt, dass diese attenuierten Viren, insbesondere auch Onyx-015 mit besonders großer Erfolgsrate bei solchen Tumoren verwendet werden kann, wo YB-1 unabhängig vom Zellzyklus im Zellkern vorkommt. Normalerweise ist YB-1 im Cytoplasma, insbesondere auch im perinukleärem Plasma vorhanden. In der S-Phase des Zellzyklus findet sich YB-1 im Zellkern von sowohl normalen wie auch Tumorzellen. Dies ist jedoch nicht ausreichend, um eine virale Onkolyse unter Verwendung der von attenuierten Adenoviren zu bewerkstelligen. Die im Stand der Technik beschriebene vergleichsweise geringe Wirksamkeit von attenuierten Adenoviren wie Onyx-015 beruht auch auf deren fehlerhaften Anwendung. Mit anderen Worten, es können derartige Systeme, insbesondere auch Onxy-015 mit einer größeren Wirksamkeit dort eingesetzt werden, wo die molekularbiologischen Voraussetzungen für eine virale Onkolyse gegeben sind. Im Falle von insbesondere Onyx-015 liegen diese Voraussetzungen vor bei solchen Tumorerkrankungen, deren Zellen eine vom Zellzyklus unabhängige Kernlokalisation von YB-1 aufweist. Diese Form der Kernlokalisation kann dabei durch die Art des Tumors selbst bedingt sein, oder aber durch die hierin beschriebenen erfindungsgemäßen Agenzien bewirkt werden. Die vorliegende Erfindung definiert somit eine neue Gruppe von Tumoren bzw. Tumorerkrankungen und damit auch von Patienten, die mit den erfindungsgemäßen Agenzien, besonders aber auch mit dem im Stand der Technik bereits beschriebenen attenuierten Adenovirus, bevorzugt mit E1B-defizienten, bevorzugterweise E1B 55 kDa-defizienten, Adenovirus und bevorzugtererweise mit Onyx-015 mit großer Erfolgsrate behandelt werden können.

Eine weitere Gruppe von Patienten, die unter Verwendung der im Stand der Technik beschriebenen Adenoviren, insbesondere solche, die E1B-defizient sind, wie beispielsweise ONYX-015, therapiert werden können, sind jene, bei denen durch Anlegen bestimmter Bedingungen gewährleistet wird, dass YB-1 in den Kern wandert oder transportiert wird. Die Verwendung derartiger Adenoviren bei dieser Patientengruppe beruht insoweit auf der Erkenntnis, dass die Induktion der viralen Replikation auf der Kernlokalisation von YB-1 mit anschließender Bindung an den E2-late-Promotor beruht. Infolge der hierin offenbarten Erkenntnisse ist das Adenovirus ONYX-015, welches E1B-defizient ist, nicht in der Lage, den Kerntransport des zellulären YB-1 zu vermitteln. Dies begründet die beschränkte Verwendung und Erfolg von ONYX-015 auf solche Tumoren, die YB-1 bereits im Kern aufweisen. Dies ist jedoch nur bei einer sehr geringen Patientengruppe gegeben. Durch Kernlokalisation von YB-1 kann durch Stress von außen bzw. lokal applizierten Stress induziert werden. Diese Induzierung kann beispielsweise durch Bestrahlung, insbesondere UV-Bestrahlung, Anwendung von Zytostatika, wie sie unter anderem hierin ebenfalls offenbart sind, und Hyperthermie erfolgen. Im Zusammenhang mit der Hyperthermie ist beachtlich, dass diese zwischenzeitlich sehr spezifisch realisiert werden kann und somit ebenfalls spezifisch ein Kerntransport von YB-1 in den Zellkern erfolgt und infolgedessen die Voraussetzungen für eine Replikation des Adenovirus und damit einer Zell- und Tumorlyse gegeben sind (Stein U, Jurchott K, Walther W, Bergmann S, Schlag PM, Royer HD. Hyperthermia-induced nuclear translocation of transcription factor YB-1 leads to enhanced expression of multidrug resistance-related ABC transporters. J Biol Chem. 2001,276(30):28562-9; Hu Z, Jin S, Scotto KW. Transcriptional activation of the MDR1 gene by UV irradiation. Role of NF-Y and Spl. J Biol Chem. 2000 Jan 28;275(4):2979-85; Ohga T, Uchiumi T, Makino Y, Koike K, Wada M, Kuwano M, Kohno K. Direct involvement of the Y-box binding protein YB-1 in genotoxic stress-induced activation of the human multidrug resistance 1 gene. J Biol Chem. 1998, 273(11):5997-6000).

Das erfindungsgemäße Medikament würde somit an solche Patienten und Patientengruppen verabreicht werden bzw. wäre für solche bestimmt, bei denen durch geeignete Vorbehandlungen ein Transport von YB-1, insbesondere in die entsprechenden Tumorzellen, bedingt werden würde.

Damit betrifft die Erfindung in einem weiteren Aspekt auch ein Verfahren zum Screenen von Patienten, die mit einem attenuierten Adenovirus, wie Onyx-1 oder allgemeiner mit E1B-defizienten, bevorzugterweise E1B 55 kDa-defizienten, Adenovirus behandelbar sind, gekennzeichnet durch die folgenden Schritte:
- Untersuchen einer Probe des Tumorgewebes und
- Festellen, ob YB-1 im Kern Zellzyklus-unabhängig lokalisiert ist.

Die Probe des Tumorgewebes kann dabei durch Punktion oder durch einen chirurgischen Eingriff erhalten werden. Die Feststellung, ob im Kern YB-1 Zellzyklus-unabhängig lokalisiert ist, wird dabei häufig unter Verwendung mikroskopischer Techniken und/oder mittels Immunhistoanalyse, typischerweise unter Verwendung von Antikörpern, erfolgen. Die Detektion von YB-1 erfolgt dabei unter Verwendung eines Mittels, das ausgewählt ist aus der Gruppe, die Antikörper gegen YB-1, umfasst. Der Nachweis darüber, dass YB-1 im Kern und insbesondere dort Zellzyklus-unabhängig lokalisiert wird, ist dem Fachmann bekannt. Beispielsweise kann bei dem Durchmustern von gegen YB-1 gefärbten Gewebeschnitten die Lokalisation von YB-1 leicht erkannt werden. Dabei ergibt sich bereits infolge der Häufigkeit des Auftretens von YB-1 im Kern, dass es sich um eine Zellzyklus-unabhängige Lokalisation im Kern handelt. Eine weitere Möglichkeit zum zellzyklusunabhängigen Nachweise von YB-1 im Kern besteht in der Durchführung einer Färbung gegen YB-1 und Feststellen, ob YB-1 im Kern lokalisiert ist, und Durchführung der Bestimmung des Zellstadiums der Zellen. Auch dies kann mittels geeigneter Antikörper erfolgen (Doppelimmunohistoanalyse).

Die Erfindung wird im folgenden an Hand der Figuren und Beispiel weiter erläutert, aus denen sich weitere Merkmale, Ausführungsformen, Anwendungen und Vorteile ergeben. Dabei zeigt
- Fig. 1: die grundsätzliche molekulargenetische Organisation des Adenovirus;
- Fig. 2: eine Übersicht verschiedener erfindungsgemäßer Nukleinsäure- bzw. Adenoviruskonstrukte; und

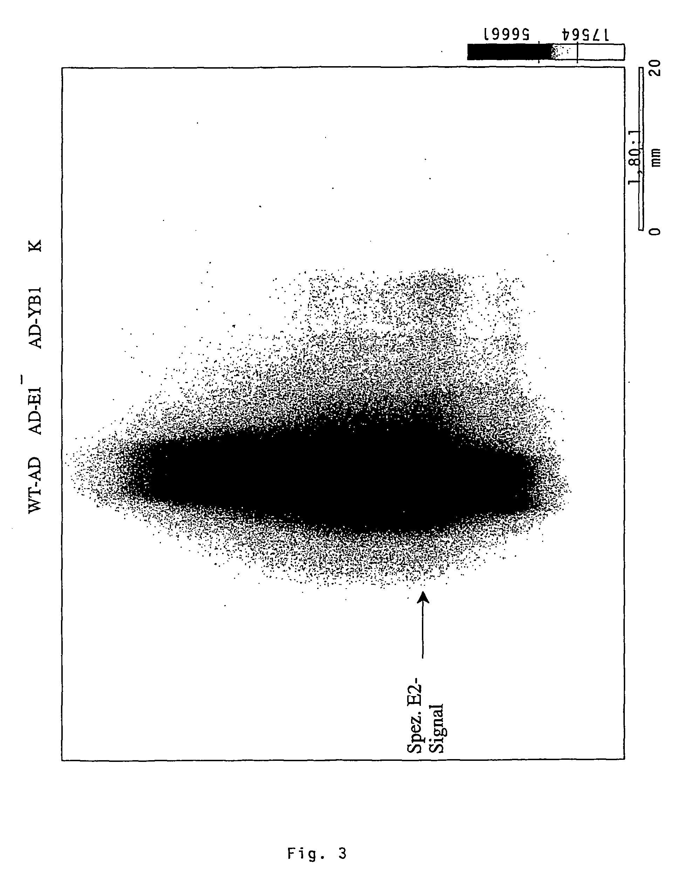

- Fig. 3: das Ergebnis einer Northern Blot-Analyse.

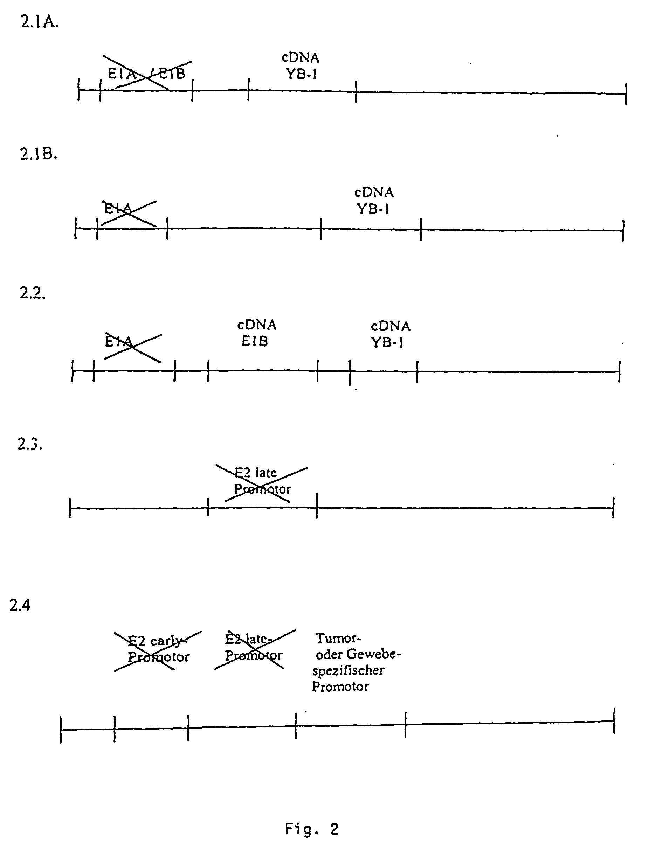

In Fig. 2 sind verschiedene der erfindungsgemäßen Nukleinsäure bzw. Nukleinsäurekonstrukte dargestellt. So zeigt Fig. 2.1A einen erfindungsgemäßen rekombinanten adenoviralen Vektor, der E1A-defizient und E1B-defizient ist, aber das Protein YB-1 exprimiert.

Fig. 2.1B zeigt einen erfindungsgemäßen rekombinanten adenoviralen Vektor, der ebenfalls YB-1 exprimiert, bei dem jedoch die E1A-Region deletiert ist. Da E1A für die Expression für E1B verantwortlich ist, kommt es zu keiner bzw. einer verringerten Aktivierung von E1B, obwohl der Vektor das Gen aufweist.

Fig. 2.2 zeigt einen erfindungsgemäßen rekombinanten adenoviralen Vektor, der ebenfalls YB-1 exprimiert. Hier wird das Gen E1B bzw. E1B-55kDa über einen externen E1A-unabhängigen Promotor gesteuert. Ein derartiger Promotor kann der CMV -, RSV- oder der YB-1-Promotor sein.

Fig. 2.3 zeigt einen weiteren erfindungsgemäßen rekombinanten adenoviralen Vektor, der einen YB-1- unabhängigen E2 late-Promotor aufweist. Dies wird dadurch erreicht, dass der vollständige E2 late-Promotor entfernt wird oder eine gezielte Veränderung der Gensequenz innerhalb des Promotors vorgenommen wird, an welche YB-1 bindet (die sogenannte Y-Box)

Fig. 2.4 zeigt einen weiteren erfindungsgemäßen rekombinanten adenoviralen Vektor, bei dem sowohl der E2 late als auch der E2 early -Promotor deletiert und somit nicht mehr funktional aktiv ist. In dem schematisch dargestellten Vektor sind die beiden Promotoren durch einen Tumor- oder Gewebe-spezifischen Promotor, wie er hierin offenbart ist, ersetzt.

### Beispiel 1: Bedeutung von YB-1 für die adenovirale Replikation

Um den Nachweis zu erbringen, dass YB-1 die Expression von E2 über den E2 late-Promotor stuert, wurde der folgende experimentelle Ansatz gewählt.

Tumorzellen (HeLa-Zellen) wurden entweder mit Wildtyp-Adenovirus, einen E1-minus Adenovirus oder mit einem E1-minus Adenovirus, welches YB-1 exprimiert (AdYB-1) infiziert (K steht dabei für Kontrolle; nicht infiziert). Nach 24 h wurde die gesamte RNA isoliert. Anschließend wurde eine Northern Blot-Analyse durchgeführt. Die isolierte RNA wurde dann gelelektrophoretisch in einem Formaldehyd-Agarose-Gel nach der Größe aufgetrennt und auf eine Nylon-Membran geblottet und unter UV fixiert. Als radioaktiv markierte Sonde wurde ein cDNA-Fragment von 250 Basen, das komplementär ist zu einer Sequenz verwendet, die zwischen dem E2 early Promotor und dem E2 late Promotor liegt. Die Markierung der Sonde erfolgte mit Hilfe des Random Prime Labeling Systems der Firma Amersham. Die Auswertung der Filme ergab, dass nur bei mit Wildtyp-Adenovirus infizierten Zellen ein E2-spezifisches Signal vorhanden ist.

### Beispiel 2: Bedeutung von YB-1 für die adenovirale Replikation

Um den Nachweis zu erbringen, dass YB-1 die Expression von E2 über den E2 late-Promotor steuert, wurde der folgende weitere experimentelle Ansatz gewählt, der auf dem in Beispiel 1 beschriebenen Ansatz aufbaut.

Die radioaktive Sonde nach Beispiel 1 wird durch Kochen für 2 Minuten in Wasser entfernt und nochmals mit einer anderen cDNA-Sonde hybridisiert. Diese Sonde liegt stromaufwärts des E2 late-Promotors.

Die Auswertung ergab das folgende Ergebnis: Sowohl die Wildtyp-Adenovirus infizierten Zellen wie auch die mit AdYB-1 infizierten Zellen weisen ein deutliches Signal bzw. eine spezifische E2-Bande auf. Daraus ergibt sich, dassYB-1 die E2-Region über den E2 late-Promotor steuert bzw. aktiviert.

### Beispiel 3: Nachweis der spezifischen Bindung von YB-1 an den E2-Promotor

Dem Experiment liegt die Überlegung zugrunde, das YB-1 als Transkriptionsfaktor an die Y-Box (CAAT-Sequenz) innerhalb des E2 late-Promotors binden sollte. Um eine solche spezifische Bindung von YB-1 an diesen Promotor nachzuweisen, wird eine sogenannte EMSA-Analyse (electrophoretic mobility shift assay) durchgeführt. Dabei werden 24 h nach Infektion der Zellen mit Wildtyp-Adenovirus die Kernprotein isoliert. Anschließend werden 1 - 10 µg Protein und ein kurzes DNA-Fragment mit einer Länge von 30 bis 80 Basen, das die E2 late-Promotorsequenz aufweist, zusammen bei 37°C für 30 Minuten inkubiert. Dieses DNA-Fragment (Oligo) wird zuvor mit einer Kinase am 5'-Ende mit ³²P radioaktiv markiert. Anschließend erfolgt die Auftrennung in einem nativen Polyacrylamidgel. Falls das Protein YB-1 an einer Sequenz am Oligo bindet, kommt es zu einem sogenannten shift (Verschiebung), da durch die Bindung von YB-1 an das kurze DNA-Fragment, das am 5'-Ende radioaktiv markiert ist, im Gel langsamer wandert als ein ungebundenes Oligo. Dieser shift kann wieder aufgehoben werden, sobald man einen hundertfachen Überschuss an nicht markiertem Oligo zum Reaktionsansatz zugibt.

Als Ergebnis konnte festgestellt werden, dass YB-1 spezifisch an den E2-late-Promotor bindet.

Als Kontrolle wurde Kommpetitions-Experimente durchgeführt. Dabei wird ein Überschuß vom nicht-markiertem E2-late-Promotor-Fragment zum Reaktionsansatz zugegeben. Daraufhin ist in dem obigen Reaktionsansatz ein shift nicht mehr festzustellen.

## Patentansprüche

1. Nukleinsäure umfassend eine adenovirale Nukleinsäure, **dadurch gekennzeichnet, dass** die Nukleinsäure eine für YB-1 codierende Nukleinsäuresequenz umfasst, wobei YB-1 ein Vertreter der Y-Box-Proteinfamilie ist.

2. Nukleinsäure nach Anspruch 1, **dadurch gekennzeichnet, dass** die adenovirale Nukleinsäure eine für E1-B codierende Nukleinsäure umfasst.

3. Nukleinsäure nach Anspruch 1 oder 2, **dadurch gekennzeichnet, dass** die adenovirale Nukleinsäure eine für E4orf6 codierende Nukleinsäure umfasst.

4. Nukleinsäure nach Anspruch 1 oder 2, **dadurch gekennzeichnet, dass** ein Promotor vorgesehen ist, der die Expression von E1-B steuert.

5. Nukleinsäure nach einem der Ansprüche 3 oder 4, **dadurch gekennzeichnet, dass** ein Promotor vorgesehen ist, der die Expression von E4orf6 steuert

6. Nukleinsäure nach einem der Ansprüche 1 bis 5, **dadurch gekennzeichnet, dass** E1B das E1-B 55 kDa-Protein ist.

7. Nukleinsäure nach einem der Ansprüche 1 bis 6, **dadurch gekennzeichnet, dass** die Nukleinsäure für funktional inaktive Genprodukte E1B und/oder E1 und/oder E3 und/oder E4 codiert.

8. Adenovirales Replikationssystem umfassen eine adenovirale Nukleinsäure, wobei die adenovirale Nukleinsäure defizient für die Expression des E1A-Proteins ist, und umfassend eine Nukleinsäure eines Helfervirus, wobei die Nukleinsäure des Helfervirus eine Nukleinsäuresequenz umfasst, die für YB-1 codiert, wobei YB-1 ein Vertreter der Y-Box-Proteinfamilie ist.

9. Adenovirales Replikationssystem nach Anspruch 8, **dadurch gekennzeichnet, dass** die adenovirale Nukleinsäure und/oder die Nukleinsäure des Helfervirus als replizierbarer Vektor vorliegt.

10. Vektor, bevorzugterweise ein Expressionsvektor, umfassend eine Nukleinsäure nach einem der Ansprüche 1 bis 7.

11. Vektorgruppe umfassend mindestens zwei Vektoren, wobei die Vektorgruppe insgesamt ein adenovirales Replikationssystem nach Anspruch 8 oder 9 umfasst.

12. Vektorgruppe nach Anspruch 11, **dadurch gekennzeichnet, dass** eine jede Komponente des adenoviralen Replikationssystems auf einem eigenen Vektor, bevorzugterweise einem Expressionsvektors, angeordnet ist.

13. Vektorgruppe nach Anspruch 11, **dadurch gekennzeichnet, dass** mindestens zwei Komponenten des adenoviralen Replikationssystems auf einem Vektor der Vektorgruppe angeordnet sind.

14. Adenovirus umfassend eine Nukleinsäure nach einem der Ansprüche 1 bis 7.

15. Adenovirus nach Anspruch 14 umfassend ein Kapsid.

16. Zelle umfassend eine Nukleinsäure nach einem der Ansprüche 1 bis 7 und/oder ein adenovirales Replikationssystem nach Anspruch 8 oder 9 und/oder einen Vektor nach Anspruch 10 und/oder eine Vektorgruppe nach einem der Ansprüche 11 bis 13 und/oder einen Adenovirus nach Anspruch 14 oder 15.

17. Verwendung einer Nukleinsäure nach einem der Ansprüche 1 bis 7 und/oder eines adenoviralen Replikationssystems nach Anspruch 8 oder 9 und/oder eines Vektors nach Anspruch 10 und/oder einer Vektorgruppe nach einem der Ansprüche 11 bis 13 und/oder eines Adenovirus nach Anspruch 14 oder 15 und/oder einer Zelle nach Anspruch 16 zur Herstellung eines Medikaments.

18. Verwendung nach Anspruch 17, **dadurch gekennzeichnet, dass** das Medikament für die Behandlung und/oder Prophylaxe von Tumorerkrankungen ist.

19. Verwendung nach Anspruch 17 oder 18, **dadurch gekennzeichnet, dass** das Medikament weiter eine pharmazeutisch wirksame Verbindung enthält.

20. Verwendung nach Anspruch 19, **dadurch gekennzeichnet, dass** die pharmazeutische Verbindung ausgewählt ist aus der Gruppe, die Zytostatika umfasst, die bevorzugterweise ausgewählt sind aus der Gruppe, die cis-Platin, Taxo1, Daunoblastin, Adriamycin und Mitoxantron umfasst.

21. Verwendung einer Nukleinsäure nach einem der Ansprüche 1 bis 7 und/oder eines adenoviralen Replikationssystems nach Anspruch 8 oder 9 und/oder eines Vektors nach Anspruch 10 und/oder einer Vektorgruppe nach einem der Ansprüche 11 bis 13 und/oder eines Adenovirus nach Anspruch 14 oder 15 und/oder einer Zelle nach Anspruch 16 zur Herstellung eines Medikaments für die Behandlung und/oder Prophylaxe von Tumorerkrankungen, wobei die Tumorzellen eine vom Zellzyklus unabhängige Kernlokalisation von YB-1 aufweisen.

22. Verwendung nach Anspruch 21, **dadurch gekennzeichnet, dass** die Nukleinsäure für eine adenovirale Nukleinsäure codiert und die adenovirale Nukleinsäure E1B-defizient, insbesondere E1B 55kDa-defizient ist.

23. Verwendung nach Anspruch 21, **dadurch gekennzeichnet, dass** der Adenovirus EIBdefizient, insbesondere E1B 55 kDa- defizient ist.

24. Verwendung nach Anspruch 21, **dadurch gekennzeichnet, dass** die Nukleinsäure für eine adenovirale Nukleinsäure codiert und die adenovirale Nukleinsäure für E1B codiert, insbesondere für E1B 55kDa.

25. Verwendung nach Anspruch 21, **dadurch gekennzeichnet, dass** der Adenovirus E1B exprimiert, insbesondere E1B 55 kDa.

26. Verwendung nach Anspruch 21, 22 oder 23, **dadurch gekennzeichnet, dass** die adenovirale Nukleinsäure für E1A codiert.

27. Verwendung nach einem der Ansprüche 21, 22 oder 23, **dadurch gekennzeichnet, dass** der Adenovirus E1A exprimiert.

28. Verwendung nach einem der Ansprüche 21 bis 27, **dadurch gekennzeichnet, dass** der Tumor und/oder die Tumorerkrankung ausgewählt ist aus der Gruppe, die p53-positive Tumoren, p53-negative Tumoren, maligne Tumoren, benigne Tumoren und Kombinationen davon umfasst.

29. Verfahren zum Screenen von Patienten, die mit einem E1B-defizienten, bevorzugterweise E1B 55 kDa-defizienten, Adenovirus behandelbar sind, **gekennzeichnet durch** die folgenden Schritte:
- Untersuchen einer Probe des Tumorgewebes und
- Festellen, ob YB-1 im Kern Zellzyklus-unabhängig lokalisiert ist, wobei YB-1 ein Vertreter der Y-Box-Proteinfamilie ist.

30. Verfahren nach Anspruch 29, **dadurch gekennzeichnet, dass** die Untersuchung des Tumorgewebes unter Verwendung eines Mittels erfolgt, das ausgewählt ist aus der Gruppe, die Antikörper gegen YB-lumfasst.

31. Verwendung eines Antikörpers gegen YB-1 zur Bestimmung von Patienten, insbesondere Tumorpatienten, die mit einem E1B-defizientem, bevorzugterweise E1B 55kDadefizientem Adenovirus behandelbar sind, wobei YB-1 ein Vertreter der Y-Box-Proteinfamilie ist.

32. Komplex umfassend mindestens ein YB-1-Molekül und mindestens ein E1B 55 kDa-Protein, wobei YB-1 ein Vertreter der Y-Box-Proteinfamilie ist.

33. Komplex nach Anspruch 32, **dadurch gekennzeichnet, dass** das YB-1-Molekül ein transgenes YB-1-Molekül ist.

34. Komplex nach Anspruch 32 oder 33, **dadurch gekennzeichnet, dass** das YB-1 nukleär exprimiertes YB-1 ist.

35. Verwendung eines E1B-defizienten Adenovirus zur Herstellung eines Medikaments zur Behandlung von Tumoren, die YB-1 im Kern aufweisen, wobei YB-1 ein Vertreter der Y-Box-Proteinfamilie ist.

36. Verwendung nach Anspruch 35, **dadurch gekennzeichnet, dass** die Kernlokalisation von YB-1 unter dem Einfluss exogener Maßnahmen erfolgt.

37. Verwendung nach Anspruch 36, **dadurch gekennzeichnet, dass** die exogenen Maßnahmen solche Maßnahmen sind, die ausgewählt sind aus der Gruppe, die Bestrahlung, Zytostatika und Hyperthermie umfasst.

38. Verwendung nach Anspruch 36 oder 37, **dadurch gekennzeichnet, dass** die exogene Maßnahme an dem Organismus angewandt wird, für den das Medikament bestimmt ist.

## Claims

1. A nucleic acid comprising an adenoviral nucleic acid, **characterized in that** the nucleic acid comprises a nucleic acid sequence coding for YB-1, whereby YB-1 is a member of the Y-Box protein family.

2. The nucleic acid according to claim 1, **characterized in that** the adenoviral nucleic acid comprises a nucleic acid coding for E1-B.

3. The nucleic acid according to claim 1 or 2, **characterized in that** the adenoviral nucleic acid comprises a nucleic acid coding for E4orf6.

4. The nucleic acid according to claim 1 or 2, **characterized in that** a promoter is provided which controls the expression of E1-B.

5. The nucleic acid according to any of claims 3 or 4, **characterized in that** a promoter is provided which controls the expression of E4orf6.

6. The nucleic acid according to any of claims 1 to 5, **characterized in that** E1-B is the E1-B 55 kDa protein.

7. The nucleic acid according to any of claims 1 to 6, **characterized in that** the nucleic acid codes for functionally inactive gene products E1B and/or E1 and/or E3 and/or E4.

8. An adenoviral replication system comprising an adenoviral nucleic acid, whereby the adenoviral nucleic acid is deficient for the expression of the E1A protein, and comprising a nucleic acid of a helper virus, whereby the nucleic acid of the helper virus comprises a nucleic acid sequence coding for YB-1, whereby YB-1 is a member of the Y-Box protein family.

9. The adenoviral replication system according to claim 8, **characterized in that** the adenoviral nucleic acid and/or the nucleic acid of the helper virus is/are present as a replicable vector.

10. A vector, preferably an expression vector, comprising a nucleic acid according to any of claims 1 to 7.

11. A vector group comprising at least two vectors, whereby the vector group comprises, in its entirety, an adenoviral replication system according to claim 8 or 9.

12. The vector group according to claim 11, **characterized in that** each component of the adenoviral replication system is arranged on a separate vector, preferably an expression vector.

13. The vector group according to claim 11, **characterized in that** at least two components of the adenoviral replication system are arranged on a vector of the vector group.

14. An adenovirus comprising a nucleic acid according to any of claims 1 to 7.

15. The adenovirus according to claim 14 comprising a capsid.

16. A cell comprising a nucleic acid according to any of claims 1 to 7 and/or an adenoviral replication system according to claim 8 or 9 and/or a vector according to claim 10 and/or a vector group according to any of claims 11 to 13 and/or an adenovirus according to claim 14 or 15.

17. Use of a nucleic acid according to any of claims 1 to 7 and/or an adenoviral replication system according to claim 8 or 9 and/or a vector according to claim 10 and/or a vector group according to any of claims 11 to 13 and/or an adenovirus according to claim 14 or 15 and/or a cell according to claim 16 for the manufacture of a medicament.

18. The use according to claim 17, **characterized in that** the medicament is for the treatment and/or prophylaxis of tumor diseases.

19. The use according to claim 17 or 18, **characterized in that** the medicament further comprises a pharmaceutically active compound.

20. The use according to claim 19, **characterized in that** the pharmaceutical compound is selected from the group comprising cytostatics, which are preferably selected from the group comprising cis-platin, taxol, daunoblastin, adriamycin and mitoxantron.

21. Use of a nucleic acid according to any of claims 1 to 7 and/or an adenoviral replication system according to claim 8 or 9 and/or a vector according to claim 10 and/or a vector group according to any of claims 11 to 13 and/or an adenovirus according to claim 14 or 15 and/or a cell according to claim 16 for the manufacture of a medicament for the treatment and/or prophylaxis of tumor diseases, whereby the tumor cells show nuclear localisation of YB-1 independent of the cell cycle.

22. The use according to claim 21, **characterized in that** the nucleic acid codes for an adenoviral nucleic acid and the adenoviral nucleic acid is E1B deficient, in particular E1B 55 kDa deficient.

23. The use according to claim 21, **characterized in that** the adenovirus is E1B deficient, in particular E1B 55 kDa deficient.

24. The use according to claim 21, **characterized in that** the nucleic acid codes for an adenoviral nucleic acid and the adenoviral nucleic acid codes for E1B, in particular E1B 55 kDa.

25. The use according to claim 21, **characterized in that** the adenovirus expresses E1B, in particular E1B 55 kDa.

26. The use according to claim 21, 22 or 23, **characterized in that** the adenoviral nucleic acid codes for E1A.

27. The use according to any of claims 21, 22 or 23, **characterized in that** the adenovirus expresses E1A.

28. The use according to any of claims 21 to 27, **characterized in that** the tumor and/or the tumor disease is/are selected from the group comprising p53-positive tumors, p53-negative tumors, malignant tumors, benign tumors and combinations thereof.

29. A method for the screening of patients who may be treated with an E1B deficient, preferably E1B 55 kDa deficient adenovirus, comprising the following steps:
- examining a sample of the tumor tissue and
- determining whether YB-1 is localised in the nucleus independent of the cell cycle, whereby YB-1 is a member of the Y-Box protein family.

30. The method according to claim 29, **characterized in that** the examination of the tumor tissue is performed by using a means selected from the group comprising antibodies against YB-1.

31. Use of an antibody directed against YB-1 for the identification of patients, in particular tumor patients, which may be treated with an E1B deficient, preferably E1B 55 kDa deficient adenovirus, whereby YB-1 is a member of the Y-Box protein family.

32. A complex comprising at least one YB-1 molecule and at least one E1B 55 kDa protein, whereby YB-1 is a member of Y-Box protein family.

33. The complex according to claim 32, **characterized in that** the YB-1 molecule is a transgenic YB-1 molecule.

34. The complex according to claim 32 or 33, **characterized in that** the YB-1 is YB-1 expressed in the nucleus.

35. Use of an E1B deficient adenovirus for the manufacture of a medicament for the treatment of tumors which have YB-1 in the nucleus, whereby YB-1 is a member of the Y-Box protein family.

36. The use according to claim 35, **characterized in that** the nuclear localisation of YB-1 occurs under the influence of exogenous measures.

37. The use according to claim 36, **characterized in that** the exogenous measures are measures selected from the group comprising irradiation, cytostatics and hyperthermia.

38. The use according to claim 36 or 37, **characterized in that** the exogenous measure is applied to an organism for which the medicament is intended.

## Revendications

1. Acide nucléique comprenant un acide nucléique adénoviral, **caractérisé en ce que** l'acide nucléique comprend une séquence d'acide nucléique codant YB-1, où YB-1 est un représentant de la famille des protéines de liaison à la boîte Y.

2. Acide nucléique selon la revendication 1, **caractérisé en ce que** l'acide nucléique adénoviral comprend un acide nucléique codant pour E1-B.

3. Acide nucléique selon la revendication 1 ou 2, **caractérisé en ce que** l'acide nucléique adénoviral comprend un acide nucléique codant pour E4orf6.

4. Acide nucléique selon la revendication 1 ou 2, **caractérisé en ce que** l'on prévoit un promoteur, qui régule l'expression de E1-B.

5. Acide nucléique selon l'une des revendications 3 ou 4, **caractérisé en ce que** l'on prévoit un promoteur, qui régule l'expression de E4orf6.

6. Acide nucléique selon l'une des revendications 1 à 5, **caractérisé en ce que** E1B est la protéine E1-B de 55 kDa.

7. Acide nucléique selon l'une des revendications 1 à 6, **caractérisé en ce que** l'acide nucléique code pour les produits géniques fonctionnellement inactifs E1B et/ou E1 et/ou E3 et/ou E4.

8. Système de réplication adénoviral, comprenant un acide nucléique adénoviral, où l'acide nucléique adénoviral est déficient pour l'expression de la protéine E1A, et comprenant un acide nucléique d'un virus assistant, où l'acide nucléique du virus assistant comprend une séquence d'acide nucléique, qui code pour YB-1, où YB-1 est un représentant de la famille des protéines de liaison à la boîte Y.

9. Système de réplication adénoviral selon la revendication 8, **caractérisé en ce que** l'acide nucléique adénoviral et/ou l'acide nucléique du virus assistant se présente sous forme de vecteur réplicable.

10. Vecteur, de préférence un vecteur d'expression, comprenant un acide nucléique selon l'une des revendications 1 à 7.

11. Groupe de vecteurs, comprenant au moins deux vecteurs, où le groupe de vecteurs comprend au total un système de réplication adénoviral selon la revendication 8 ou 9.

12. Groupe de vecteurs selon la revendication 11, **caractérisé en ce que** chaque composant du système de réplication adénoviral est disposé sur un vecteur propre, de préférence un vecteur d'expression.

13. Groupe de vecteurs selon la revendication 11, **caractérisé en ce qu'**au moins deux composants du système de réplication adénoviral sont disposés sur un vecteur du groupe de vecteurs.

14. Adénovirus comprenant un acide nucléique selon l'une des revendications 1 à 7.

15. Adénovirus selon la revendication 14 comprenant une capside.

16. Cellule comprenant un acide nucléique selon l'une des revendications 1 à 7, et/ou un système de réplication adénoviral selon la revendication 8 ou 9, et/ou un vecteur selon la revendication 10, et/ou un groupe de vecteurs selon l'une des revendications 11 à 13, et/ou un adénovirus selon la revendication 14 ou 15.

17. Utilisation d'un acide nucléique selon l'une des revendications 1 à 7, et/ou d'un système de réplication adénoviral selon la revendication 8 ou 9, et/ou d'un vecteur selon la revendication 10, et/ou d'un groupe de vecteurs selon l'une des revendications 1 à 13, et/ou d'un adénovirus selon la revendication 14 ou 15, et/ou d'une cellule selon la revendication 16, pour la fabrication d'un médicament.

18. Utilisation selon la revendication 17, **caractérisée en ce que** le médicament est destiné au traitement et/ou à la prophylaxie des affections tumorales.

19. Utilisation selon la revendication 17 ou 18, **caractérisée en ce que** le médicament comprend en outre un composé pharmaceutiquement efficace.

20. Utilisation selon la revendication 19, **caractérisée en ce que** le composé pharmaceutique est choisi dans le groupe comprenant les cytostatiques, qui sont choisis de préférence dans le groupe, comprenant le cis-platine, le taxol, la daunoblastine, l'adriamycine et le mitoxanthron.

21. Utilisation d'un acide nucléique selon l'une des revendications 1 à 7, et/ou d'un système de réplication adénoviral selon la revendication 8 ou 9, et/ou d'un vecteur selon la revendication 10, et/ou d'un groupe de vecteurs selon l'une des revendications 11 à 13, et/ou d'un adénovirus selon la revendication 14 ou 15, et/ou d'une cellule selon la revendication 16, pour la fabrication d'un médicament destiné au traitement et/ou à la prophylaxie des affections tumorales, où les cellules tumorales présentent une localisation nucléaire de YB-1 indépendante du cycle cellulaire.

22. Utilisation selon la revendication 21, **caractérisée en ce que** l'acide nucléique code pour un acide nucléique adénoviral, et l'acide nucléique adénoviral est déficient en E1-B, en particulier déficient en E1B de 55kDa.

23. Utilisation selon la revendication 21, **caractérisée en ce que** l'adénovirus est déficient en E1-B, en particulier déficient en E1B de 55 kDa.

24. Utilisation selon la revendication 21, **caractérisée en ce que** l'acide nucléique code un acide nucléique adénoviral, et l'acide nucléique adénoviral code pour E1B, en particulier E1B de 55 kDa.

25. Utilisation selon la revendication 21, **caractérisée en ce que** l'adénovirus exprime E1B, en particulier E1B de 55 kDa.

26. Utilisation selon la revendication 21, 22 ou 23, **caractérisée en ce que** l'acide nucléique adénoviral code pour E1A.

27. Utilisation selon la revendication 21, 22 ou 23, **caractérisée en ce que** l'adénovirus exprime E1A.

28. Utilisation selon l'une des revendications 21 à 27, **caractérisée en ce que** la tumeur et/ou l'affection tumorale est choisie dans le groupe comprenant les tumeurs p53-positives, les tumeurs p53-négatives, les tumeurs malignes, les tumeurs bénignes et leurs combinaisons.

29. Procédé de criblage de patients, qui peuvent être traités avec un adénovirus déficient en E1B, de préférence déficient en E1B de 55 kDa, **caractérisé par** les étapes suivantes :
- analyse d'un échantillon de tissu tumoral et
- détermination si YB-1 est localisé dans le noyau indépendamment du cycle cellulaire, YB-1 étant un représentant de la famille des protéines de liaison à la boîte Y.

30. Procédé selon la revendication 29, **caractérisé en ce que** l'analyse du tissu tumoral se fait en utilisant un agent, qui est choisi dans le groupe constitué des anticorps contre YB-1.

31. Utilisation d'un anticorps contre YB-1 pour détecter des patients, en particulier des patients atteints d'une tumeur, qui peuvent être traités avec un adénovirus déficient en E1B, de préférence déficient en EIB de 55 kDa, YB-1 étant un représentant de la famille des protéines de liaison à la boîte Y.

32. Complexe comprenant au moins une molécule YB-1 et au moins une protéine de 55 kDa de E1B, YB-1 étant un représentant de la famille des protéines de liaison à la boîte Y.

33. Complexe selon la revendication 32, **caractérisé en ce que** la molécule YB-1 est une molécule YB-1 transgénique.

34. Complexe selon la revendication 32 ou 33, **caractérisé en ce que** le YB-1 est un YB-1 exprimé au niveau nucléaire.

35. Utilisation d'un adénovirus déficient en E1B pour la fabrication d'un médicament pour le traitement des tumeurs, qui présentent YB-1 dans le noyau, YB-1 étant un représentant de la famille des protéines de liaison à la boîte Y.

36. Utilisation selon la revendication 35, **caractérisée en ce que** la localisation nucléaire de YB-1 s'effectue sous l'influence de mesures exogènes.

37. Utilisation selon la revendication 36, **caractérisée en ce que** les mesures exogènes sont des mesures qui sont choisies dans le groupe constitué de l'irradiation, des cytostatiques et de l'hyperthermie.

38. Utilisation selon la revendication 36 ou 37, **caractérisée en ce que** la mesure exogène est appliquée sur l'organisme pour lequel est destiné le médicament.
